(19) Europäisches Patentamt
European Patent Office
Office européen des brevets

(11) **EP 1 917 869 A1**

(12) **EUROPEAN PATENT APPLICATION**

(43) Date of publication:
**07.05.2008 Bulletin 2008/19**

(21) Application number: **07018207.6**

(22) Date of filing: **17.09.2007**

(51) Int Cl.:
*A23L 1/30* (2006.01)　　　*A23L 1/308* (2006.01)
*A23L 1/0522* (2006.01)　　　*A23L 1/305* (2006.01)
*A61K 35/74* (2006.01)

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK RS**

(30) Priority: **19.09.2006 US 845652 P
05.07.2007 US 773729**

(71) Applicant: **National Starch and Chemical
Investment Holding
Corporation
New Castle,
Delaware 19720 (US)**

(72) Inventors:
• **Brown, Ian Lewis
Gymea Bay, NSW 2227 (AU)**
• **Birkett, Anne M.
Sommerville,
New Jersey 08876 (US)**
• **Le Leu, Richard
Manningham,
South Australia 5086 (AU)**
• **Graeme P. Young
Malvern,
South Australia 5061 (AU)**

(74) Representative: **Held, Stephan et al
Meissner, Bolte & Partner
Postfach 86 03 29
81630 München (DE)**

(54) **Probiotic/non-probiotic combinations**

(57)　This invention pertains to a method of promoting gastrointestinal health by ingesting a composition comprising one or more probiotic microorganisms, one or more carbohydrate sources, and one or more resistant protein products. Large intestinal health is benefited by one or more of the following: increased fecal weight, lower fecal pH, increased carbohydrate fermentation, and amelioration of protein fermentation.

EP 1 917 869 A1

**Description**

This application claims priority to provisional application U.S. S.N. 60/845,652 filed September 19, 2006.

FIELD OF THE INVENTION

This invention relates to a method of promoting gastro-intestinal health by ingesting a composition comprising one or more probiotic microorganisms, one or more carbohydrate sources, and one or more resistant protein products.

BACKGROUND OF THE INVENTION

Starch products are an important and major component of the diet. Starch, a complex carbohydrate, is composed of two types of polysaccharide molecules, amylose, a mostly linear and flexible polymer of D-anhydroglucose units that are linked by alpha-1,4-D-glucosidic bonds, and amylopectin, a branched polymer of linear chains that are linked by alpha-1,6-D-glucosidic bonds. Starch is digested predominantly in the small intestine by the enzyme alpha-amylase. Alpha-amylase hydrolyzes alpha-1,4-D-glucosidic bonds, but does not hydrolyze the alpha-1,6-D-glucosidic linkages, resulting in less complete hydrolysis of the amylopectin fraction.

Studies have shown the further importance to the diet of starch and fiber. The consumption of dietary fiber is particularly important to digestive health, and has been implicated as being useful for the prevention or treatment of certain diseases such as colon cancer. Generally, dietary fiber is defined to be the polysaccharides and remnants of plant materials that are resistant to hydrolysis (digestion) by human alimentary enzymes, and may include such components as nonstarch polysaccharides, lignin and minor components such as waxes, cutin and suberin. Because of the potential health benefits of foods rich in dietary fiber, many countries have recommended the increased consumption of such foods as a part of their dietary guidelines.

It is known that some naturally occurring starches and certain starch processing operations can result in the transformation of starch into starch that is resistant to pancreatic amylase, known simply as resistant starch. Resistant starch resists digestion by digestive amylases, such as pancreatic alpha-amylase and absorption in the small intestine, but passes into the large intestine where it is fermented by colonic microflora to mainly short chain fatty acids and gases. Research literature indicates that this fermentation of resistant starch by colonic microorganisms has numerous beneficial effects including improving colonic health and reducing the chances of developing diseases such as diverticulosis and colon cancer. Further, as it is not utilized until it reaches the large intestine, where it is fermented to short chain fatty acids, resistant starch has a reduced caloric value and in respect of these properties has the benefits of dietary fiber.

Resistant starch (RS) has been classified in the literature into four categories depending on the causes of resistance. RS1 is a physically inaccessible starch due to entrapment of granules within a protein matrix or within a plant cell wall. RS2 is a granular starch that resists digestion by pancreatic alpha-amylase. RS3 is a retrograded, nongranular starch formed by heat/moisture treatment of starch or starch foods. RS4 is a chemically modified starch that resists digestion by alpha-amylase and includes starch esters (eg acetylated), starch ethers (eg hydroxypropylated), cross-linked starches (eg sodium trimetaphosphate and sodium tripolyphosphate), etc,.

Various methods have been reported for producing various types of resistant starch products, including those disclosed in U.S. Patent Nos. 5,593,503 and 6,664,389.

Proteins are another major and important part of the diet. Composed of amino acids, proteins are used by the body for growth, maintenance and general well-being. Proteins may be of either animal or plant origin, providing a number of functional roles in foods and feeds. They are important nutrients and can contribute functionally, such as to both flavor and color.

Studies have shown that digestion of all proteins is not the same. Some proteins may resist digestion and absorption in the small intestine, passing into the large intestine where they can be utilized by colonic microflora as a source of nitrogen. The presence of certain resistant proteins may alter the relative proportions of bacterial species.

The gastrointestinal tract microflora of the healthy subject protects the host from pathogen invasion. In the young, the elderly and the health status compromised patient, however, this protective barrier is less effective. An individual can have their health status compromised to various degrees ranging from minor stress and related events, for example, dietary changes, emotional and nutritional stresses, to extreme cases such as in immuno-compromised patients and patients undergoing radiological (radiation) and/or chemo-therapy.

Probiotic microorganisms have been described to exert antimicrobial effects which refers to the actions of the probiotic preparation on another microbe or group of microbes in the gastrointestinal tract. These are directly applicable to the use of probiotics for enhanced resistance against intestinal pathogenic microorganisms and the pathogens that they can produce. The types of interactions include competitive colonisation as well as adhesion and growth inhibition.

Competitive colonisation refers to the fact that the beneficial probiotic strain can successfully out-compete the pathogen for either available nutrients or for colonisation sites on the colonic epithelium. Since many gastrointestinal pathogens attach to the intestinal mucosa as the first step in infection, it would be beneficial to the host if this adhesion could be inhibited. There are reports that lactobacilli produce components which inhibit attachment of enterotoxigenic Escherichia coli to intestinal mucosa. In addition, various compounds produced during growth of the probiotic have been shown to inhibit pathogen growth. These include organic acids such as lactic and acetic acid, reuterin and bacteriocins. Organic acids lower the pH and thereby can indirectly affect growth of the pathogen. In addition, the lactic and acetic acids can be toxic to microbes. Reuterin which inhibits the growth of a very broad range of cells is produced by Lactobacillus reuteri when grown in the presence of glycerol. Numerous bacteriocins have been reported to be produced by lactobacilli e.g. Acidophilin, Acidolin, Lactocidin, Bacteriocin, Bulgarican, Lactolin, Lactobacillin and Lactobrevin. They can either have a very broad range of activity or alternatively specifically inhibit the growth of a very limited range of closely related microbes. For example, Lactobacillus sp. can exhibit specific antagonistic effects towards Clostridium ramnosum.

There are different levels of specific bacterial populations in the various regions of the gastrointestinal tract of humans and animals. In addition, it has been shown that the specific strains of the various genera and species vary from one region of the digestive tract to another.

It is the contention of many scientists that the health and well-being of people can be positively or negatively influenced by the microorganisms which inhabit the gastrointestinal tract, and in particular, the large intestine. These microorganisms, through the production of toxins, metabolic by-products, short chain fatty acids, and the like affect the physiological condition of the host.

The constitution and quantity of the gut microflora can be influenced by conditions or stress induced by disease, life style, travel, and other factors. If microorganisms which positively affect the health and well-being of the individual can be encouraged to populate the large bowel, this should improve the physiological well-being of the host.

The introduction of beneficial microorganisms, or probiotics, is normally accomplished by the ingestion of the organisms in drinks, yoghurts, capsules, and other forms in such a way that the organism arrives in a viable condition in the large bowel.

Surprisingly, it has now been discovered that gastointestinal health may be benefited by ingesting a composition comprising one or more probiotic microorganisms, one or more carbohydrate sources, and one or more resistant protein products.

SUMMARY OF THE INVENTION

This invention pertains to a method of promoting gastrointestinal health by ingesting a composition comprising one or more probiotic microorganisms, one or more carbohydrate sources, and one or more resistant protein products. Large intestinal health is benefited by one or more of the following: increased fecal weight, lower fecal pH, increased carbohydrate fermentation, and amelioration of protein fermentation.

As used herein, the term carbohydrate source is intended to mean high amylose carbohydrates, high amylose resistant starch products, and fructo-oligosaccharides including without limitation fructo-oligosaccharides such as fructo-, galacto-, malto-, isomalto-, gentio-, xylo-, palatinose-, soybean- (includes raffinose and stachyose), chito-, agaro-, neoagaro-, $\alpha$-gluco-, $\beta$-gluco-, cyclo-inulo-, glycosylsucrose, lactulose, lactosucrose and xylsucrose; and polysaccharides such as high amylose starch and flour.

The term resistant starch (RS), as used herein, is defined as the sum of starch and starch degradation products that are not absorbed in the small intestine of healthy individuals and may be measured by a variety of tests known in the art. As used herein, resistant starch is measured by treatment with pancreatic alpha-amylase in the test described, infra.

The term resistant starch product, as used herein, is defined as a product which contains 5-100% resistant starch by weight.

Normal amylose, as used herein, is intended to mean amylose which contains between 250 and 12,500 D-anhydroglucose units and a gel permeation chromatography ("GPC") peak molecular weight of about 200,000.

As used herein, low molecular weight amylose is intended to mean substantially linear polymers containing from about 30 to 250 anhydroglucose units and a GPC peak molecular weight of about 15,000 primarily linked by alpha-1,4-D-glucosidic bonds.

As used herein, high amylose is intended to mean a product containing at least about 27% amylose for wheat or rice flour and at least about 50% amylose for other sources, by weight of its starch as measured by the potentiometric method detailed in the Examples section.

As used herein, resistant protein is intended to mean the sum of protein and protein degradation products that are not absorbed in the small intestine of healthy individuals and may be measured by a variety of tests known in the art. As used herein, resistant protein is measured by the test described, infra.

The term resistant protein product, as used herein, is defined as a product which contains 5-100% resistant protein by weight.

As used herein, probiotic bacteria or microorganisms are intended to mean, a live microbial feed supplement which beneficially affects the host mammal by improving its intestinal microbial balance. This is the definition provided by R. Fuller (AFRC Institute of Food Research, Reading Laboratory, UK) in--Journal of Applied Bacteriology, 1989. 66, pp.365-378. "Probiotics in Man and Animals--A Review".

As used herein, a prebiotic composition is an at least partially nondigestible food ingredient that beneficially affects the host by selectively stimulating the growth, activity or both of one or a limited number of species of microorganisms already resident in the colon.

As used herein, a synbiotic composition may be a yogurt, capsule or other form of introduction into the host mammal, including human beings, in which prebiotics are used in combination with a live microbial food supplement. The live microbial food supplement beneficially affects the host animal by improving its intestinal microbial balance.

As used herein, gastrointestinal is intended to include the stomach, small intestine, and large intestine. Large intestine is intended to include the colon and rectum, and in humans, is intended to include the colon, rectum and caecum.

DETAILED DESCRIPTION OF THE INVENTION

This invention pertains to a method of promoting gastrointestinal health by ingesting a composition comprising one or more probiotic microorganisms, one or more carbohydrate sources, and one or more resistant protein products. Large intestinal health is benefited by one or more of the following: increased fecal weight, lower fecal pH, increased carbohydrate fermentation, and amelioration of (decreased) protein fermentation.

Probiotic bacteria or microorganisms are intended to include one or more species which beneficially affects the host mammal after ingestion and may include any species known in the art including without limitation Bifiobacterium such as Bif. Pseudolongum, Bif. Lactis, Bif. Longum, Bif. Infantis, and Bif. bifidum; Bacteroids such as Bact. Vulgatus and Bact. Fragilis, Clostridium such as Cl. butyricum; Eubacteria; Fusobacterium; Propionibacterium; Streptococcus; Enterococcus; Lactococcus; Staphylococcus; Peptostreptococcus; and Lactobacillus such as Lactobacillus acidophilus. Probiotic microorganisms also include yeasts such as Saccharomyces. The invention is not, however, limited to these particular microorganisms. In one embodiment, the probiotic microorganism is Bifiobacterium.

The carbohydrate source used may be any carbohydrate source known in the art, including without limitation starches, flours, grains, legumes, polysaccharides and oligosaccharides. The carbohydrate source is selected from high amylose carbohydrates, high amylose resistant starch products, and fructo-oligosaccharides. In an embodiment, the carbohydrate source is one which at least partially resists digestion and is fermented, such that butyrate is produced. In one aspect of the invention, the carbohydrate source used is a resistant starch product, including RS1, RS2, RS3 and RS4 types. In another aspect of the invention, the carbohydrate source is a high amylose resistant starch product. In yet another aspect of the invention, the carbohydrate source is a fructo-oligosaccharide.

The resistant starch product may be one found in nature or one made using starches found in nature. A native starch as used herein, is one as it is found in nature. Also suitable are starches derived from a plant obtained by standard breeding techniques including crossbreeding, translocation, inversion, transformation or any other method of gene or chromosome engineering to include variations thereof. In addition, starch derived from a plant grown from induced mutations and variations of the above generic composition which may be produced by known standard methods of mutation breeding are also suitable herein.

Typical sources for the starches are cereals, tubers, roots, legumes and fruits. The native source can include corn (maize), pea, potato, sweet potato, banana, barley, wheat, rice, sago, amaranth, tapioca, arrowroot, canna, or sorghum, as well high amylopectin or high amylose varieties thereof. As used herein, the term "high amylopectin" is intended to include a starch containing at least about 90, particularly at least about 95, more particularly at least about 98% amylopectin by weight. As used herein, the term "high amylose" is intended to include a starch containing at least about 27% amylose for wheat or rice flour and at least about 50% amylose for other sources, particularly at least about 70, more particularly at least about 80% amylopectin by weight.

In one embodiment, the carbohydrate source used is extracted from a plant source having an amylose extender genotype, the starch of such material comprising less than about 10% by weight amylopectin. This material is derived from a plant breeding population, particularly corn, which is a genetic composite of germplasm selections and comprises at least 75% by weight normal amylose, and in one embodiment at least 85% normal amylose as measured by butanol fractionation/exclusion chromatography techniques. The starch further comprises less than about 10%, by weight, and in one embodiment less than 5%, amylopectin, and additionally from about 8 to 25% low molecular weight amylose. The starch is extracted from the grain of a starch bearing plant having a recessive amylose extender genotype coupled with numerous amylose extender modifier genes. This starch and the method of preparation are described in U.S. Pat. No. 5,300,145, which is incorporated herein by reference.

In one embodiment, the carbohydrate source used is a high amylose resistant starch product. In one embodiment, the resistant starch product is a chemically, physically and/or enzymatically treated or modified starch.

In another embodiment, the resistant starch product used is one containing the RS2 type of resistant starch, including those modified by heat-moisture treatment to increase the percent resistant starch by weight of the product, those typically found in high amylose carbohydrate materials and/or those described in US Patent Nos. 5,593,503 and 5,902,410. In yet another embodiment, the carbohydrate source used is a resistant starch product containing the RS3 type of resistant starch, including those described in US Patent Nos. 5,281,276 and 5,409,542. In a further embodiment, the carbohydrate source used is a resistant starch product containing the RS4 type of resistant starch, including chemically modified starches selected from the group consisting of oxidation, crossbonding, etherification, esterification, acidification, dextrinisation, and mixtures thereof and those described in US Patent No. 5,855,946. In a still further embodiment, the degree of enzyme susceptibility of the resistant starch is modified by altering the conformation or structure of the starch, such as by acid or enzyme thinning and cross bonding using difunctional reagents. In yet another embodiment, the amylolysis of the high amylose resistant starch is modified to give starch granules characterised by pits or erosions which can extend from the surface to the interior of the granules. These pits allow the entry of enzymes to the more enzyme susceptible core of the starch granule which is solubilized. Low or normal amylose containing starches can also provide a source of RS, particularly if they are consumed in their native form or if they have been modified in some manner (such as by physical, hydrothermal, enzymatic, acidic, chemical, etc.).

In a still further embodiment, the carbohydrate source used is an oligosaccharide and in a yet further embodiment, the carbohydrate source is a fructo-oligosaccharide. Fructo-oligosaccharides suitable for the present invention may include any fructo-oligosaccharides available for consumption. Commercial fructo-oligosaccharides presently available include without limitation fructo-, galacto-, malto-, isomalto-, gentio-, xylo-, palatinose-, soybean- (includes raffinose and stachyose), chito-, agaro-, neoagaro-, .alpha.-gluco-, .beta.-gluco-, cyclo-inulo-, glycosylsucrose, lactulose, lactosucrose and xylsucrose. It will be appreciated by one skilled in the art, however, that other oligosaccharides would also be suitable for inclusion in the composition of the present invention.

The protein source may be any resistant protein product, including those of single or multiple cell animal or vegetable origin. Resistant protein products include without limitation proteins sourced from meat, fish, maize, rice, wheat, oats, millet, peas, peanuts, soy, sago, cassava, arrowroot, taro, and beans. In one embodiment, the resistant protein product is of vegetable origin. In another embodiment, the protein source is synthetic, including oligomethionine. In yet another embodiment, the resistant starch product is derived from a cereal source and in yet another from potato. The source of protein may also be in the form of flour, extracts or isolates.

In one aspect of the invention, the probiotic microorganism is a Bifidobacteria, the carbohydrate source is a high amylose corn (maize) resistant starch, and the resistant protein product is potato protein in the form of an isolate.

Each component of the composition may be present in any ratio. In one embodiment, the probiotic is present in an amount of from about $10^4$ - $10^{12}$ CFU of probiotic microorganisms per 10 g of composition, and in one embodiment from about $10^6$ - $10^{11}$ CFU of probiotic microorganisms per 10 g of probiotic microorganism/carbohydrate source/resistant protein composition and in yet another embodiment from about $10^8$ - $10^{10}$ CFU of probiotic microorganisms per 10 g of probiotic microorganism/carbohydrate source/resistant protein composition. In one embodiment, the carbohydrate source is present in an amount of from about 0.5g to about 9.5g per 10g of probiotic microorganism/carbohydrate source/resistant protein composition, and in one embodiment is present in an amount of from about 1g to about 8g per 10g of probiotic microorganism/carbohydrate source/resistant protein composition, and in yet another embodiment from about 5g to about 7g per 10g of probiotic microorganism/carbohydrate source/resistant protein composition. In one embodiment, the resistant protein is present in an amount of from about 0.5g to about 9.5g per 10g of probiotic microorganism/carbohydrate source/resistant protein composition, and in one embodiment is present in an amount of from about 1g to about 6g per 10g of probiotic microorganism/carbohydrate source/resistant protein composition, and in yet another embodiment from about 2g to about 4g per 10g of probiotic microorganism/carbohydrate source/resistant protein composition.

The composition may be ingested by mammals, including humans, to promote gastrointestinal health, in one embodiment large intestinal health and in another distal intestinal health. Large intestinal health is benefited by one or more of the following: increased fecal weight, lower fecal pH, increased carbohydrate fermentation, and reduced protein fermentation. Lower fecal pH is due to increased short chain fatty acid content, particularly acetate and butyrate as well as decreased ammonia. Carbohydrate fermentation is preferred over protein fermentation as the by-products of carbohydrate fermentation are primarily beneficial while the by-products of protein fermentation are not. Ingestion of the composition also resulted in highest butyrate to ammonia ratio and highest fecal weight to cresol ratio. The composition may be used to prevent and/or treat gastrointestinal cancer, hepatic encephalopathy, prostate cancer, constipation, diarrhea, inflammatory bowel disease, irritable bowel disease, irritable bowel syndrome, diverticular disease, hemhorroids, osteoporosis, bone fractures, insulin resistance and insulin sensitivity. The composition may also be used to increase bone mineral density, and control insulin, glucose and mineral bioavailability/balances.

The composition of this invention may be used in any food or beverage product (hereinafter collectively referred to as foods). In one embodiment, the food is a cultured dairy product such as yogurts, cheeses, and sour creams. The composition may be added at any amount which is acceptable to the consumer from both an organoleptic and a physiological standpoint and in one embodiment is used in an amount of from about 0.1 to 50%, and in another embodiment in an amount of from about 1 to 25%, by weight of the food.

EP 1 917 869 A1

The composition may also be used in a pharmaceutical or nutritional product, including but not limited to synbiotic compositions, diabetic foods and supplements, dietetic foods, foods to control glycemic response and glycemic index, and tablets and other pharmaceutical dosage forms.

The composition may also be used in a feed for livestock or companion animals.

EMBODIMENTS

The following embodiments are presented to further illustrate and explain the present invention and should not be taken as limiting in any regard.

1. A composition comprising a probiotic microorganism, a carbohydrate source, and a resistant protein product, wherein the carbohydrate source is selected from the group consisting of a high amylose carbohydrate, a high amylose resistant starch product, and a fructo-oligosaccharide.

2. A two part composition comprising a first part which contains two components selected from the group comprising a probiotic microorganism, a carbohydrate source, and a resistant protein product and a second component comprising the remaining component.

3. A three part composition comprising a first part which comprises a probiotic microorganism, a second part which comprises a carbohydrate source, and a third part which comprises a resistant protein product.

4. The composition of embodiment 1, wherein the probiotic microorganism is selected from the group consisting of Bifiobacterium , Bacteroids, Clostridium, Eubacteria, Fusobacterium, Propionibacterium, Streptococcus, Enterococcus, Lactococcus, Staphylococcus, Peptostreptococcus, Lactobacillus, and Saccharomyces.

5. The composition of embodiment 1, wherein the probiotic microorganism is Bifidobacterium.

6. The composition of embodiment 1, wherein the resistant starch product is a high amylose resistant starch product.

7. The composition of embodiment 6, wherein the high amylose resistant starch product is a chemically, physically and/or enzymatically treated or modified starch.

8. The composition of embodiment 7, wherein the resistant starch product is a starch chemically modified, wherein the chemical is selected from the group consisting of oxidation, crossbonding, etherification, esterification, acidification, dextrinisation, and mixtures thereof.

9. The composition of embodiment 7, wherein the resistant starch product is a starch which has been physically modified by heat-moisture treatment to increase the percent resistant starch by weight of the product.

10. The composition of embodiment 1, wherein the resistant protein product is a potato protein.

11. The composition of embodiment 1, wherein the probiotic microorganism is a Bifidibacteria, the carbohydrate source is a high amylose resistant starch, and the resistant protein product is potato protein.

12. The composition of embodiment 1 or 11, wherein the probiotic microorganism is present in an amount of from $10^4$ to $10^{12}$ CFU, the carbohydrate source is present in an amount of from about 0.5 g to 9.5 g, and the resistant protein product is present in an amount of from about 0.5g to 9.5g per 10 g of probiotic microorganism/carbohydrate source/resistant protein composition.

13. The composition of embodiment 1 or 11, wherein the probiotic microorganism is present in an amount of from $10^8$ to $10^{10}$ CFU, the carbohydrate source is present in an amount of from about 5 g to 7 g, and the resistant protein product is present in an amount of from about 2g to 4g per 10 g probiotic microorganism/carbohydrate source/resistant protein composition.

14. The composition of embodiment 1 or 11, wherein the composition is selected from the group consisting of a food product, a beverage product, a pharmaceutical product and a nutritional product.

15. A method of promoting gastrointestinal health comprising ingesting the composition of embodiment 1 or 11.

16. A method of promoting distal large intestinal health comprising ingesting the composition of embodiment 1 or 11.

17. The method of embodiment 16, wherein fecal weight is increased, fecal pH is lowered, carbohydrate fermentation is increased, and/or protein fermentation is decreased.

18. A method of increasing the amount of short chain fatty acids produced in the gastrointestinal tract comprising ingesting the composition of embodiment 1 or 11.

19. A method of increasing the amount of short chain fatty acids selected from the group consisting of acetate, propionate, and butyrate produced in the gastrointestinal tract comprising ingesting the composition of embodiment 1 or 11.

20. A method of increasing the amount of butyrate produced in the gastrointestinal tract comprising ingesting the composition of embodiment 1 or 11.

21. A method of increasing fecal weight comprising ingesting the composition of embodiment 1 or 11.

22. A method of preventing or curing a condition selected from the group consisting of gastrointestinal cancer, hepatic encephalopathy, prostrate cancer, constipation, diarrhea, inflammatory bowel disease, irritable bowel disease, irritable bowel syndrome, diverticular disease, hemhorroids, osteoporosis, bone fractures, insulin resistance and insulin sensitivity comprising ingesting the composition of embodiment1 or 11.

23. A method of increasing bone mineral density comprising ingesting the composition of embodiment 1 or 11.

24. A method of controlling or balancing insulin, glucose or/and mineral bioavailability comprising ingesting the composition of embodiment 1 or 11.

EXAMPLES

The following examples are presented to further illustrate and explain the present invention and should not be taken as limiting in any regard. All parts and percentages are given by weight and all temperatures in degrees Celsius (°C) unless otherwise noted.

The following ingredients were used throughout the examples.

1) resistant starch product from high amylose corn, where the resistant starch content was 65%, as is basis (commercially available from National Starch and Chemical Company, USA).

2) resistant protein product isolated from potato, where the resistant protein content was 16.0% (commercially available from Hokuren Co. Japan); prepared from potato juice by steam coagulation after adjustment of pH to 5.0-5.5

3) Bifidobacterium lactis (LAFTI B94), $10^{10}$ CFU/g, (commercially available from DSM Specialties, Australia)

The following test procedures were used throughout the examples.

A. Simulated Digestion - (Modified from Englyst et al., European Journal of Clinical Nutrition, 1992, 46,S33-S50) - Food samples are ground/minced as if masticated. Powder starch samples are screened to a particle size of 250 microns or less. A 500-600 mg $\pm$ 0.1 mg of sample is weighed and added to the sample tube. 10 ml of a pepsin (0.5%), guar gum (0.5%), and HCl (0.05 M) solution is added to each tube.

Blank and glucose standard tubes are prepared. The blank is 20 ml of a buffer containing 0.25 M sodium acetate and 0.02% calcium chloride. Glucose standards are prepared by mixing 10 ml sodium acetate buffer (described above) and 10ml of 50 mg/ml glucose solution. Standards are prepared in duplicate.

The enzyme mix is prepared by adding 18 g of porcine pancreatin (Sigma P-7545) to 120 ml of deionized water, mixing well, then centrifuging at 3000g for 10 minutes. The supernatant is collected and 48mg of dry invertase (Sigma I-4504) and 0.5 ml AMG E (Novo Nordisk) are added.

The sample tubes are pre-incubated at 37°C for 30 min, then removed from the bath and 10 ml of sodium acetate buffer is added along with glass balls/marbles (to aid in physical breakdown of the sample during shaking).

5 ml of the enzyme mixture is added to the samples, blank, and standards. The tubes are shaken horizontally in a 37°C waterbath at approximately 180 strokes/min. Time "zero" represents the first addition of the enzyme mixture to the first tube.

After 120 minutes, a 0.5-ml aliquot is removed from the incubating samples and placed into a separate tube of 20ml 66% ethanol (to stop the reaction). After 1 hour, an aliquot is centrifuged at 3000g for 10 minutes.

The glucose concentration in the tube is measured using the glucose oxidase/peroxidase method (Megazyme *Glucose Assay Procedure* GLC9/96). This is a colorimetric procedure.

The degree of starch digestion is determined by calculating the glucose concentration against the glucose standards, using a conversion factor of 0.9. The amount of resistant starch is determined by subtracting the % starch digested (dry weight basis) at 120 minutes from 100%.

Every sample analysis batch includes a reference sample of uncooked cornstarch. The accepted range of % digestion values for cornstarch are:

| Time (minutes) | 20 | 120 |
|---|---|---|
| Sample 1 (control)[1] | 18 $\pm$ 3 | 80 $\pm$ 5 |
| [1]Melogel® starch, cornstarch commercially available from National Starch and Chemical Company, Bridgewater, NJ, USA. Thus, cornstarch would contain about 20% resistant starch. | | |

B. Amylose content by potentiometric titration

0.5 g of a starch (1.0 g of a ground grain) sample was heated in 10mls of concentrated calcium chloride (about 30% by weight) to 95°C for 30 minutes. The sample was cooled to room temperature, diluted with 5 mls of a 2.5% uranyl acetate solution, mixed well, and centrifuged for 5 minutes at 2000 rpm. The sample was then filtered to give a clear solution. The starch concentration was determined polarimetrically using a 1 cm polarimetric cell. An aliquot of the sample (normally 5 mls) was then directly titrated with a standardized 0.01 N iodine solution while recording the potential using a platinum electrode with a KCI reference electrode. The amount of iodine needed to reach the inflection point was measured directly as bound iodine. The amount of amylose was calculated by assuming 1.0 gram of amylose will bind with 200 miligrams of iodine.

C. Measurement of protein digestibility

Protein digestibility was determined in vivo with Wistar rats, using casein as a reference protein. Rats were housed individually in metal screened cages, and fed one of three diets for 10 days - protein-free diet, 10% protein isolate diet, and 10% casein diet. Food intake and body weight was recorded daily, and feces were collected for the last 3 days of the experimental period. Dietary nitrogen intake and fecal nitrogen were measured.

$$\text{Resistant protein (\%)} = 100 - \text{true protein digestibility (\%)}$$

where

$$\text{True protein digestibility (\%)} = [\text{nitrogen intake} - (\text{fecal nitrogen excreted} -$$

$$\text{fecal nitrogen excreted on the protein-free diet})] / \text{nitrogen intake X 100}$$

Composition of the experimental diet.

| Ingredients | Protein Free Diet | 10% Casein protein diet | 10% Protein isolate diet |
| --- | --- | --- | --- |
| Cornstarch | 902.5 | 802.5 | 802.5 |
| Casein | | 100 | |
| Protein isolate | | | 100 |
| Corn oil | 50 | 50 | 50 |
| Mineral mix * | 35 | 35 | 35 |
| Vitamin mix * | 10 | 10 | 10 |
| Choline bitartrate | 2.5 | 2.5 | 2.5 |
| * based on AIN 76 (AIN = American Institute of Nutrition) Note: AIN is a standard rat diet upon which many dietary manipulations are made for rat feeding studies and well known in the art. | | | |

D. Rat study. Effect of a probiotic/non-probiotic combination. Male Sprague-Dawley rats were housed in plastic cages with wire bottom screens. 12 rats per group were fed one of 8 different diets for a period of 4 weeks. The diets were a modified AIN-76 diet (see the table below), consisting of a no-fibre diet, or the same diet to which the three components were added either individually, as a two-part combination, or as a three-part combination. The protein isolate replaced casein (at 19% of diet), the high amylose resistant starch product replaced regular cornstarch (at 10% of the diet), and the probiotic was added at 1 % of the diet.

| Ingredient | Diet 1 - No fiber |
| --- | --- |
| | |
| Casein | 950 |
| Cornstarch | 2357.5 |
| Sucrose | 547.5 |
| Corn oil | 900 |

(continued)

| Ingredient | Diet 1 - No fiber |
|---|---|
| Minerals | 175 |
| Vitamins | 50 |
| Methionine | 15 |
| Choline | 5 |
| | |
| Total | 5000 |

At the end of the experimental period, fresh fecal samples were collected from each rat for 3 days. On the final day each rat was sacrificed, and the large intestine removed, sectioned, and cleaned for subsequent analysis. Large intestinal contents were also removed for subsequent analysis.

Measured parameters included: (1) Feces - weight, pH, short chain fatty acids (SCFA) including total, acetate, propionate and butyrate, and ammonia; and (2) urine - cresol (see table below).

Rats eating the diet with the three-part combination (Diet 8) displayed higher fecal weight and lower fecal pH than rats eating no, one or two parts of the combination. In addition ratios of fecal butyrate:ammonia, fecal weight: ammonia and fecal weight:urine cresol were highest for the three-part combination. Concentrations of total short chain fatty acids, as well as acetate and butyrate were high for the three-part combination. Overall, diet 8 (three-part combination) showed greater impact on the parameters measured than for any part of the combination.

| | Diet 1 No fibre | Diet 2 Probiotic | Diet 3 Resistant Protein | Diet 4 Resistant Protein + Probiotic | Diet 5 RS | Diet 6 RS + Probiotic | Diet 7 RS + Resistant Protein | Diet 8 RS + Resistant Protein + Probiotic |
|---|---|---|---|---|---|---|---|---|
| Fecal Weight g/d | 0.47 ± 0.03 | 0.41 ± 0.09 | 1.48 ± 0.13 | 1.41 ± 0.11 | 1.2 ± 0.3 | 0.85 ± 0.11 | 1.97 ± 0.17 | 2.51 ± 0.27 |
| Fecal pH | 7.29 ± 0.06 | 7.48 ± 0.07 | 6.92 ± 0.05 | 6.98 ± 0.04 | 6.51 ± 0.07 | 6.41 ± 0.06 | 6.36 ± 0.05 | 6.20 ± 0.03 |
| Fecal Total SCFA umol/g | 32.8 ± 5.5 | 26.1 ± 3.5 | 26.1 ± 3.6 | 27.7 ± 3.4 | 37.8 ± 7.0 | 51.8 ± 6.7 | 50.1 ± 7.0 | 51.5 ± 6.7 |
| Fecal Acetate umol/g | 21.0 ± 3.6 | 16.9 ± 2.5 | 16.0 ± 2.5 | 16.6 ± 2.5 | 26.3 ± 5.8 | 35.8 ± 5.3 | 28.6 ± 4.5 | 36.0 ± 5.3 |
| Fecal Butyrate umol/g | 3.7 ± 0.7 | 2.9 ± 0.4 | 4.1 ± 0.4 | 4.9 ± 0.4 | 3.0 ± 0.4 | 5.0 ± 0.8 | 11.5 ± 1.6 | 11.2 ± 0.9 |
| Fecal Ammonia umol/g | 18.9 ± 1.7 | 15.0 ± 1.4 | 23.1 ± 2.2 | 24.7 ± 1.7 | 21.6 ± 2.3 | 18.1 ± 2.5 | 27.7 ± 2.7 | 18.3 ± 1.7 |
| Urine Cresol ug/ml | 59.6 ± 13.4 | 26.3 ± 3.7 | 110.5 ± 14.6 | 196.9 ± 36.0 | 106.7 ± 21.5 | 50.23 ± 12.5 | 215.0 ± 47.2 | 118.6 ± 38.4 |
| Fecal SCFA: Ammonia | 1.74 | 1.74 | 1.13 | 1.12 | 1.75 | 2.86 | 1.81 | 2.81 |

(continued)

| | Diet 1 No fibre | Diet 2 Probiotic | Diet 3 Resistant Protein | Diet 4 Resistant Protein + Probiotic | Diet 5 RS | Diet 6 RS + Probiotic | Diet 7 RS + Resistant Protein | Diet 8 RS + Resistant Protein + Probiotic |
|---|---|---|---|---|---|---|---|---|
| Fecal Butyrate: Ammonia | 0.20 | 0.19 | 0.18 | 0.20 | 0.14 | 0.28 | 0.42 | 0.61 |
| Fecal Weight: Ammonia | 0.025 | 0.027 | 0.064 | 0.057 | 0.056 | 0.047 | 0.071 | 0.137 |
| Fecal SCFA: Cresol | 0.55 | 0.99 | 0.24 | 0.14 | 0.35 | 1.03 | 0.23 | 0.43 |
| Fecal Butyrate: Cresol | 0.062 | 0.110 | 0.037 | 0.025 | 0.028 | 0.0995 | 0.053 | 0.094 |
| Fecal Weight: Cresol | 0.0079 | 0.016 | 0.013 | 0.0072 | 0.011 | 0.017 | 0.0092 | 0.021 |

**Claims**

1. A composition comprising a probiotic microorganism, a carbohydrate source, and a resistant protein product, wherein the carbohydrate source is selected from the group consisting of a high amylose carbohydrate, a high amylose resistant starch product, and a fructo-oligosaccharide.

2. A two part composition comprising a first part which contains two components selected from the group comprising a probiotic microorganism, a carbohydrate source, and a resistant protein product and a second component comprising the remaining component.

3. A three part composition comprising a first part which comprises a probiotic microorganism, a second part which comprises a carbohydrate source, and a third part which comprises a resistant protein product.

4. The embodiment of any one of claims 1-3, wherein the probiotic microorganism is selected from the group consisting of Bifiobacterium , Bacteroids, Clostridium, Eubacteria, Fusobacterium, Propionibacterium, Streptococcus, Enterococcus, Lactococcus, Staphylococcus, Peptostreptococcus, Lactobacillus, and Saccharomyces.

5. The composition of claim 4, wherein the probiotic microorganism is Bifidobacterium.

6. The composition of any one of claims 1-5, wherein the resistant starch product is a high amylose resistant starch product.

7. The composition of claim 6, wherein the high amylose resistant starch product is a chemically, physically and/or enzymatically treated or modified starch.

8. The composition of claim 7, wherein the resistant starch product is a starch chemically modified, wherein the chemical is selected from the group consisting of oxidation, crossbonding, etherification, esterification, acidification, dextrinisation, and mixtures thereof.

9. The composition of claim 7, wherein the resistant starch product is a starch which has been physically modified by heat-moisture treatment to increase the percent resistant starch by weight of the product.

**10.** The composition of any one of claims 1-9, wherein the resistant protein product is a potato protein.

**11.** The composition of any one of claims 1-10, wherein the probiotic microorganism is a Bifidibacteria, the carbohydrate source is a high amylose resistant starch, and the resistant protein product is potato protein.

**12.** The composition of any of claims 1-11, wherein the probiotic microorganism is present in an amount of from $10^4$ to $10^{12}$ CFU, the carbohydrate source is present in an amount of from about 0.5 g to 9.5 g, and the resistant protein product is present in an amount of from about 0.5g to 9.5g per 10 g of probiotic microorganism/carbohydrate source/ resistant protein composition.

**13.** The composition of any of claims 1-11, wherein the probiotic microorganism is present in an amount of from $10^8$ to $10^{10}$ CFU, the carbohydrate source is present in an amount of from about 5 g to 7 g, and the resistant protein product is present in an amount of from about 2g to 4g per 10 g probiotic microorganism/carbohydrate source/resistant protein composition.

**14.** The composition of any of claims 1-11, wherein the composition is selected from the group consisting of a food product, a beverage product, a pharmaceutical product and a nutritional product.

**15.** A method of promoting gastrointestinal health comprising ingesting the composition of any of claims 1-11.

**16.** A method of promoting distal large intestinal health comprising ingesting the composition of any of claims 1-11.

**17.** The method of embodiment 16, wherein fecal weight is increased, fecal pH is lowered, carbohydrate fermentation is increased, and/or protein fermentation is decreased.

**18.** A method of increasing the amount of short chain fatty acids produced in the gastrointestinal tract comprising ingesting the composition of any of claims 1-11.

**19.** A method of increasing the amount of short chain fatty acids selected from the group consisting of acetate, propionate, and butyrate produced in the gastrointestinal tract comprising ingesting the composition of any of claims 1-11.

**20.** A method of increasing the amount of butyrate produced in the gastrointestinal tract comprising ingesting the composition of any of claims 1-11.

**21.** A method of increasing fecal weight comprising ingesting the composition of any of claims 1-11.

**22.** A method of preventing or curing a condition selected from the group consisting of gastrointestinal cancer, hepatic encephalopathy, prostrate cancer, constipation, diarrhea, inflammatory bowel disease, irritable bowel disease, irritable bowel syndrome, diverticular disease, hemhorroids, osteoporosis, bone fractures, insulin resistance and insulin sensitivity comprising ingesting the composition of any of claims 1-11.

**23.** A method of increasing bone mineral density comprising ingesting the composition of any of claims 1-11.

**24.** A method of controlling or balancing insulin, glucose or/or mineral bioavailability comprising ingesting the composition of any of claims 1-11.

**EP 1 917 869 A1**

<table>
<tr><td colspan="4">European Patent Office</td></tr>
</table>

**European Patent Office** **EUROPEAN SEARCH REPORT**

Application Number

EP 07 01 8207

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 221 350 B1 (BROWN IAN L [AU] ET AL) 24 April 2001 (2001-04-24) * column 1, line 19 - line 25; claims * * column 1, line 66 - column 2, line 51; claims; tables * ----- | 1-24 | INV. A23L1/30 A23L1/308 A23L1/0522 A23L1/305 A61K35/74 |
| X | US 6 348 452 B1 (BROWN IAN L [AU] ET AL) 19 February 2002 (2002-02-19) * claims; examples 1,3,6 * ----- | 1-24 | |
| X | US 6 783 780 B1 (DE JONG PATRICIA [NL] ET AL) 31 August 2004 (2004-08-31) * column 4, line 60 - column 5, line 7; claims 1,5-8,13; examples II,V * ----- | 1-24 | |
| X | EP 1 228 699 A (NUTRINOVA GMBH [DE]) 7 August 2002 (2002-08-07) * paragraphs [0008], [0026] - [0029]; table 1 * ----- | 1-14 | |
| X | MORITA TATSUYA ET AL: "Resistant proteins alter cecal short-chain fatty acid profiles in rats fed high amylose cornstarch" JOURNAL OF NUTRITION, vol. 128, no. 7, July 1998 (1998-07), pages 1156-1164, XP002463539 ISSN: 0022-3166 * abstract * * page 1157, left-hand column, paragraph 3 - page 1158, left-hand column, paragraph 4; tables 1-5 * ----- -/-- | 1-24 | TECHNICAL FIELDS SEARCHED (IPC) A23L A61K |

The present search report has been drawn up for all claims

| Place of search | Date of completion of the search | Examiner |
|---|---|---|
| Munich | 7 January 2008 | Rinaldi, Francesco |

EPO FORM 1503 03.82 (P04C01)

**European Patent Office**

**EUROPEAN SEARCH REPORT**

Application Number

EP 07 01 8207

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MORITA TATSUYA ET AL: "Oligo-L-methionine and resistant protein promote cecal butyrate production in rats fed resistant starch and fructooligosaccharide" JOURNAL OF NUTRITION, vol. 129, no. 7, July 1999 (1999-07), pages 1333-1339, XP002463540 ISSN: 0022-3166 * abstract * * tables * | 1-24 | |
| A | WO 99/11542 A (TEXEL [FR]; GOSSNER JOSEF [DE]; ZIMMERMANN KURT [DE]) 11 March 1999 (1999-03-11) * page 1, paragraph 1; claims; figures * | 2,3 | |
| | | | TECHNICAL FIELDS SEARCHED (IPC) |

The present search report has been drawn up for all claims

| Place of search | Date of completion of the search | Examiner |
|---|---|---|
| Munich | 7 January 2008 | Rinaldi, Francesco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 07 01 8207

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2008

| Patent document cited in search report | | Publication date | Patent family member(s) | | Publication date |
|---|---|---|---|---|---|
| US 6221350 | B1 | 24-04-2001 | AT | 281174 T | 15-11-2004 |
| | | | WO | 9734615 A1 | 25-09-1997 |
| | | | CA | 2249361 A1 | 25-09-1997 |
| | | | DE | 69731461 D1 | 09-12-2004 |
| | | | DE | 69731461 T2 | 27-10-2005 |
| | | | DK | 888118 T3 | 14-03-2005 |
| | | | EP | 0888118 A1 | 07-01-1999 |
| | | | ES | 2234002 T3 | 16-06-2005 |
| | | | JP | 2000506870 T | 06-06-2000 |
| | | | NZ | 331950 A | 28-02-2000 |
| US 6348452 | B1 | 19-02-2002 | WO | 9734591 A1 | 25-09-1997 |
| | | | CA | 2249189 A1 | 25-09-1997 |
| | | | EP | 0901371 A1 | 17-03-1999 |
| | | | JP | 2001503016 T | 06-03-2001 |
| US 6783780 | B1 | 31-08-2004 | AT | 368469 T | 15-08-2007 |
| | | | AT | 263568 T | 15-04-2004 |
| | | | AU | 774614 B2 | 01-07-2004 |
| | | | AU | 1896600 A | 26-06-2000 |
| | | | CA | 2353544 A1 | 15-06-2000 |
| | | | DE | 69916319 D1 | 13-05-2004 |
| | | | DE | 69916319 T2 | 25-05-2005 |
| | | | DK | 1481682 T3 | 05-11-2007 |
| | | | EP | 1137424 A1 | 04-10-2001 |
| | | | ES | 2216611 T3 | 16-10-2004 |
| | | | HK | 1041208 A1 | 10-09-2004 |
| | | | HK | 1071703 A1 | 28-09-2007 |
| | | | ID | 29924 A | 25-10-2001 |
| | | | JP | 2002531510 T | 24-09-2002 |
| | | | NL | 1010770 C2 | 13-06-2000 |
| | | | WO | 0033854 A1 | 15-06-2000 |
| | | | NZ | 512253 A | 26-09-2003 |
| EP 1228699 | A | 07-08-2002 | AU | 1476402 A | 08-08-2002 |
| | | | DE | 10105305 A1 | 14-08-2002 |
| | | | JP | 2002262779 A | 17-09-2002 |
| | | | KR | 20020069475 A | 04-09-2002 |
| | | | US | 2002156046 A1 | 24-10-2002 |
| | | | ZA | 200200726 A | 02-08-2002 |
| WO 9911542 | A | 11-03-1999 | AU | 1145199 A | 22-03-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 84565206 P
- US 5593503 A
- US 6664389 B
- US 5300145 A
- US 5902410 A
- US 5281276 A
- US 5409542 A
- US 5855946 A

**Non-patent literature cited in the description**

- Probiotics in Man and Animals--A Review. **R. FULLER.** Journal of Applied Bacteriology. 1989, vol. 66, 365-378
- **ENGLYST et al.** *European Journal of Clinical Nutrition,* 1992, vol. 46, S33-S50